# EUROPÄISCHE PATENTSCHRIFT

(11) **EP 3 791 175 B1**
(45) Veröffentlichungstag und Bekanntmachung des Hinweises auf die Patenterteilung: **22.01.2025**
(21) Anmeldenummer: 19723376.0
(22) Anmeldetag: 08.05.2019
(51) Int. Cl.: G01N 33/00

(54) **MESSSTATION, EINGERICHTET ZUM MESSEN DER LUFTQUALITÄT**
MEASURING STATION, CONFIGURED TO MEASURE AIR QUALITY
STATION DE MESURE CONÇUE POUR MESURER LA QUALITÉ DE L'AIR

(30) Priorität: 09.05.2018 DE 102018111095
(43) Veröffentlichungstag der Anmeldung: 17.03.2021
(73) Patentinhaber: Westnetz GmbH, 44139 Dortmund (DE)
(72) Erfinder: BOOS, Nico, 56761 Gamlen (DE); KOKOT, Marcel, 42657 Solingen (DE); KRATZKE, Ina Sophie, 45131 Essen (DE); KRINGS, Henning, 44137 Dortmund (DE); PETERS, Verena, 44791 Bochum (DE)
(74) Vertreter: Cohausz & Florack
(86) Internationale Anmeldenummer: PCT/EP2019/061828
(87) Internationale Veröffentlichungsnummer: WO 2019/215236

(56) Entgegenhaltungen:
- CN-A- 104 678 063
- CN-U- 205 333 597
- CN-U- 206 505 064

## Beschreibung

Die Erfindung betrifft eine Messstation, eingerichtet zum Messen der Luftqualität, für die Freiluftaufstellung im städtischen Bereich.

Derartige Messstationen sind grundsätzlich bekannt in Form von Messcontainern, in denen die Messelektronik installiert ist und auf deren Dach Messgeräte zur Bestimmung der Luftqualität, beispielsweise des Feinstaubgehaltes der Luft, installiert sind. Derartige Messstationen befinden sich typischerweise an stark befahrenen Straßen oder in anderweitig durch Schadstoffquellen belasteten innerstädtischen Bereichen.

Diese konventionellen Messcontainer haben verschiedene Nachteile. Die Messcontainer sind sehr groß, blockieren daher erheblichen öffentlichen Raum und stellen ein erhebliches Sichthindernis dar, so dass insbesondere die Aufstellung an Straßenkreuzungen problematisch ist. Zudem sind die Messcontainer typischerweise recht unansehnlich und werden deshalb nur ungern im Stadtbereich, insbesondere im Innenstadtbereich, oder im Bereich städtischer Grünflächen oder in Erholungsbereichen aufgestellt, an denen eine Überprüfung der Luftqualität jedoch wünschenswert wäre. Darüber hinaus verunreinigen derartige Container schnell. Insbesondere lagern sich an stark mit Feinstaub belasteten Standorten erhebliche Mengen Feinstaub auf dem Containerdach, die durch Windstöße immer wieder aufgewirbelt werden. Dies führt auch zu Verfälschungen bei den Feinstaubmessungen. Weiterhin ermöglichen konventionelle Container nicht, sich lokal über die Luftqualität zu informieren.

Die CN 104 678 063 offenbart eine Baum-artige Messstation für die Luftqualität mit Sensoren für verschiedene Luftqualität-Indikatoren und mit an Blättern angeordneten Leuchtelementen, die abhängig von der Luftqualität angesteuert werden.

Vor diesem Hintergrund liegt der vorliegenden Erfindung die Aufgabe zugrunde, eine Messstation zum Messen der Luftqualität für die Freiluftaufstellung im städtischen Bereich zur Verfügung zu stellen, mit denen die oben genannten Nachteile zumindest teilweise vermieden werden.

Diese Aufgabe wird erfindungsgemäß gelöst durch eine Messstation, eingerichtet zum Messen der Luftqualität, für die Freiluftaufstellung im städtischen Bereich mit einer Standbasis zum Aufstellen der Messstation auf einem Untergrund, mit einer Stamm-artigen Struktur, die sich von der Standbasis in die Höhe erstreckt. Die Messstation verfügt über eine Kronen-artige Struktur am oberen Ende der Stamm-artigen Struktur, wobei mindestens ein Messsensor derart in die Messstation integriert ist, dass mit dem Messsensor Messwerte einer Messgröße für die Luftqualität der Luft im Bereich der Kronen-artigen Struktur messbar sind. Weiterhin weist die Kronen-artige Struktur mehrere hohle Kugel-förmige Elemente auf, die die äußere Form der Kronen-artigen Struktur vorgeben, und es sind Beleuchtungsmittel vorgesehen und zur Beleuchtung mindestens eines, vorzugsweise mehrerer der Kugel-förmigen Elemente angeordnet, wobei die Beleuchtungsmittel in den hohlen Kugel-förmigen Elementen angeordnet sind. Zudem ist eine Steuereinrichtung vorgesehen, die dazu eingerichtet ist, die Beleuchtungsmittel abhängig von den mit dem mindestens einen Messsensor gemessenen Messwerten zu steuern.

Auf diese Weise wird eine optisch ansprechende Messstation für den Innenstadtbereich zur Verfügung gestellt, mit der die Luftqualität der Umgebungsluft gemessen werden kann. Durch die dreiteilige Struktur der Messstation mit der Standbasis, der Stamm-artigen und der Kronen-artigen Struktur ahmt die Messstation das Erscheinungsbild eines Laubbaums nach und lässt sich auf diese Weise gut in das Stadtbild integrieren ohne als Fremdkörper zu wirken wie zum Beispiel die klobigen konventionellen Messcontainer.

Die Standbasis dient zum Aufstellen der Messstation auf einem Untergrund und verleiht der Messstation den nötigen Halt. Das Gewicht der Standbasis ist, beispielsweise durch Vorsehen entsprechender Gewichte, vorzugsweise derart bemessen, dass die Messstation ohne weitere Bodenverankerung sicher aufgestellt werden kann. Vorzugsweise ist das Gewicht so bemessen, dass die Messstation einer vorgegebenen Windgeschwindigkeit von vorzugsweise mindestens 100 km/h widerstehen kann ohne Umzufallen. Alternativ oder zusätzlich können an der Standbasis auch Befestigungselemente, zum Beispiel Befestigungsringe, zur Verankerung der Messstation am Boden vorgesehen sein.

Die Stamm-artige Struktur erstreckt sich von der Standbasis in die Höhe und hält die Kronen-artige Struktur am oberen Ende der Stamm-artigen Struktur. Durch die die Kronen-artige Struktur tragende Stamm-artige Struktur wird das Sichtfeld durch die Messstation in typischer Augenhöhe von Passanten oder Kraftfahrzeuginsassen nur minimal beeinträchtigt gegenüber einem monolithischen Messcontainer. Die Stamm-artige Struktur kann beispielsweise durch ein Metallrohr gebildet werden. Ein solches Metallrohr verleiht der Messstation Stabilität und kann die Kronen-artige Struktur sicher tragen.

Im Bereich der Kronen-artigen Struktur sind vorzugsweise ein oder mehrere Lufteinlässe vorgesehen, durch die Luft im Bereich der Kronen-artigen Struktur in das Innere der Messstation eingeleitet und dem mindestens einen Messsensor zugeführt werden kann, um die Luftqualität der Luft zu bestimmen. Um die Luft durch die ein oder mehreren Lufteinlässe einzusaugen, können in der Messstation ein oder mehrere Ansaugmittel wie zum Beispiel Ventilatoren vorgesehen sein.

Mindestens ein Lufteinlass ist vorzugsweise an der Unterseite der Kronen-artigen Struktur angeordnet und vorzugsweise nach unten gerichtet. Auf diese Weise wird der Lufteinlass vor Regen geschützt und kann Luft aus dem Bereich der Unterseite der Kronen-artigen Struktur in die Messstation einlassen, so dass Luft aus dem Bereich einer typischen Kopfhöhe von Passanten analysiert werden kann. Weiterhin hat ein nach unten angeordneter Lufteinlass den Vorteil, dass sich im Bereich um den Lufteinlass kein Feinstaub ansammeln kann wie es zum Beispiel auf dem Dach konventioneller Messcontainer der Fall wäre. Auf diese Weise kann zum Beispiel eine Feinstaubmessung mit größerer Genauigkeit durchgeführt werden.

Als Messgrößen für die Luftqualität kommen insbesondere Konzentrationen von einem oder mehreren Bestandteilen der Luft in Frage. Insbesondere können die Konzentrationen gasförmiger Bestandteile wie zum Beispiel von Kohlenmonoxid (CO), Kohlendioxid (CO₂), Stickoxiden (NOₓ), Ozon (O₃) und/oder Wasserdampf (H₂O) in der Luft gemessen werden. Zusätzlich oder alternativ können auch Konzentrationen von flüssigen oder festen Bestandteilen gemessen werden, insbesondere Feinstaub-Konzentrationen (zum Beispiel Konzentrationen von grobem, feinem oder ultrafeinem Feinstaub). Entsprechend ist mindestens ein Messsensor, insbesondere der mindestens eine Messsensor, vorzugsweise dazu eingerichtet, Messwerte einer oder mehrerer der folgenden Messgrößen zu messen: NOₓ-Konzentration, O₃-Konzentration, COz-Konzentration, Feinstaub-Konzentration oder Luftfeuchtigkeit der Luft. Insbesondere können auch mehrere Messsensoren vorgesehen sein, um Messwerte verschiedener der zuvor genannten Messgrößen zu messen.

Zusätzlich können an der Messstation auch weitere Sensoren zur Messung von Messwerten für weitere Umweltdaten vorgesehen sein, beispielsweise Temperatur-, Niederschlags-, Windrichtungs- und/oder Windgeschwindigkeits-, UV- oder auch Lautstärke-Sensoren. Diese Sensoren können beispielsweise innerhalb der Kronen-artigen Struktur oder außerhalb, insbesondere auf der Kronen-artigen Struktur angeordnet sein. Außerhalb, insbesondere auf der Kronen-artigen Struktur können insbesondere ein UV-Sensor, ein Niederschlags-Sensor, ein Windrichtungs- und/oder Windgeschwindigkeits-Sensor und/oder ein Temperatur-Sensor angeordnet sein. Auf diese Weise können neben der Luftqualität auch andere, insbesondere die Gesundheit beeinflussende Umweltbedingungen gemessen werden.

Die Höhe der Stamm-artigen Struktur ist vorzugsweise so gewählt, dass zwischen der Oberkante der Standbasis und der Unterkante der Kronen-artigen Struktur ein Abstand von mindestens 2 m vorgesehen ist. Auf diese Weise wird die Kronen-artige Struktur vor Vandalismus geschützt, da sie von einem Menschen von der Standbasis nicht unmittelbar erreichbar ist. Zur Vermeidung von Vandalismus ist die Stamm-artige Struktur vorzugsweise ohne Verzweigungen zwischen Standbasis und Kronenartiger Struktur ausgebildet, um ein Hinaufklettern an der Stamm-artigen Struktur zu erschweren. Vorzugsweise ist die Oberfläche der Stamm-artigen glatt ausgebildet, beispielsweise lackiert.

Nachfolgend werden verschiedene Ausführungsformen der Messstation beschrieben, wobei die einzelnen Ausführungsformen untereinander kombinierbar sind.

Bei einer Ausführungsform ist der Messsensor in die Standbasis integriert und es ist eine von der Kronen-artigen Struktur durch die Stamm-artige Struktur zur Standbasis verlaufende Luftleitung zur Versorgung des Messsensors mit Luft aus dem Bereich der Kronen-artigen Struktur vorgesehen. Die Integration des Messsensors in die Standbasis ermöglicht eine einfachere Wartung und einen einfacheren Zugriff auf den Messsensor, beispielsweise durch eine dafür vorgesehene, vorzugsweise verschließbare Revisionsklappe. Weiterhin ist es bei schweren Messsensoren vorteilhaft, diese in der Standbasis unterzubringen, um die Standfestigkeit der Messstation zu erhöhen.

Alternativ oder zusätzlich können ein oder mehrere Messsensoren auch direkt in der Kronen-artigen Struktur untergebracht werden, wobei in diesem Fall weitere Komponenten wie zum Beispiel Steuer- oder Auswerteelektronik auch in die Standbasis integriert sein können.

Die Stamm-artige Struktur dient bei den zuvor beschriebenen Ausführungsformen zum Hindurchführen von Kabeln und/oder Luftleitungen, um die Lufteinlässe im Bereich der Kronen-artigen Struktur mit den Messsensoren in der Standbasis oder die Messsensoren in der Kronen-artigen Struktur mit einer Steuer- oder Auswerteelektronik oder mit einer Stromversorgung in der Standbasis zu verbinden. In der Standbasis können zusätzlich Aufnahmen für weitere Messsensorik oder Auswerteelektronik vorgesehen sein, so dass sich die Messstation modular um weitere Messsensorik oder Auswerteelektronik erweitern lässt.

Bei einer weiteren Ausführungsform weist die Messstation einen Energiespeicher zur Versorgung der Messstation mit elektrischer Energie auf, der vorzugsweise in die Standbasis integriert ist. Bei dem Energiespeicher kann es sich insbesondere um eine Batterie, insbesondere um eine Auto- oder Lkw-Batterie handeln, beispielsweise mit einer Nennspannung von 12V oder 24V. Auf diese Weise kann die Messstation autark und unabhängig von sonstiger elektrischer Versorgung betrieben werden. Dies ermöglicht den mobilen Einsatz der Messstation zum Beispiel auch an Orten, an denen kein geeigneter elektrischer Netzanschluss zur Verfügung steht. Zusätzlich oder alternativ kann die Messstation jedoch auch einen Anschluss zum Anschließen an einen externen Energiespeicher, eine externe Energiequelle oder an das Stromnetz aufweisen. Weiterhin kann die Messstation zur Energieversorgung, insbesondere zum Aufladen des Energiespeichers, ein Solarmodul aufweisen. Das Solarmodul kann insbesondere auf der Kronen-artigen Struktur angeordnet werden. Auf diese Weise kann die Abschattung des Solarmoduls reduziert werden. Gleichzeitig ist das Solarmodul von unten weniger sichtbar und stört das Laubbaum-artige Gesamterscheinungsbild der Messstation nicht.

Bei einer weiteren Ausführungsform weist die Standbasis eine wasserundurchlässige Hülle auf. Auf diese Weise werden die darin enthaltene Messelektronik, Auswerteelektronik, Steuerungselektronik und/oder ein vorgesehener Energiespeicher vor Witterung geschützt. Die Kronen-artige Struktur weist vorzugsweise einen Wasserablauf auf, damit zum Beispiel durch einen Lüftungseinlass eingedrungenes Regenwasser abfließen kann. Insbesondere können an der Unterseite der Kronen-artige Struktur Wasserauslässe vorgesehen sein.

Weiterhin weist die Standbasis vorzugsweise eine fest verschlossene Hülle auf, um die darin enthaltenen Komponenten vor Vandalismus oder anderen Beschädigungen zu schützen.

Bei einer weiteren Ausführungsform weist die Standbasis eine Sitzgelegenheit auf, insbesondere eine Sitzbank. Zum Beispiel kann in die Standbasis ein Sitz oder eine Sitzbank integriert sein oder die Dimensionen der Standbasis können so gewählt sein, dass sie als Sitzgelegenheit dienen können. Auf diese Weise wird durch die Messstation gleichzeitig ein städtisches Möbelstück geschaffen, das sich dadurch noch besser in das Stadtbild integrieren lässt.

Bei einer weiteren Ausführungsform ist an der Stamm-artigen Struktur ein Lufteinlass vorgesehen, und mindestens ein Messsensor ist derart in die Messstation integriert, dass mit dem Messsensor Messwerte einer Messgröße für die Luftqualität der durch den Lufteinlass einströmenden Luft messbar sind. Durch das Vorsehen eines Lufteinlasses an der Stamm-artigen Struktur kann an einer zusätzlichen Höhe unterhalb der Kronen-artigen Struktur die Luftqualität bestimmt werden. Auf diese Weise lässt sich beispielsweise ein Höhenprofil der Luftqualität, zum Beispiel der Feinstaub- oder Ozonkonzentration, ermitteln. Dies kann beispielsweise sinnvoll sein, um Belastungen (zum Beispiel durch Feinstaub oder Ozon) für verschiedene Personengruppen (Fußgänger, Radfahrer, Kinder etc.) feststellen zu können.

Zusätzlich oder alternativ kann auch an der Standbasis ein Lufteinlass vorgesehen sein und mindestens ein Messsensor derart in die Messstation integriert sein, dass mit dem Messsensor Messwerte einer Messgröße für die Luftqualität der durch den Lufteinlass einströmenden Luft messbar sind. Auf diese Weise kann die Luftqualität in Bodennähe gemessen werden. Weist die Standbasis beispielsweise eine Sitzgelegenheit auf, so kann der Lufteinlass an der Standbasis im Bereich der Sitzgelegenheit angeordnet sein, um die Luftqualität im Bereich der Sitzgelegenheit zu messen.

Erfindungsgemäß weist die Kronen-artige Struktur mehrere Kugel-förmige Elemente, vorzugsweise von verschiedener Größe, auf, die die äußere Form der Kronen-artigen Struktur vorgeben. Beispielsweise können die Kugel-förmigen Elemente untereinander und/oder an einer gemeinsamen Haltestruktur befestigt sein. Die Kugel-förmigen Elemente können auch stoffschlüssig miteinander verbunden sein und ein einheitliches Bauteil bilden.

Das Vorsehen derartiger Kugel-förmiger Elemente hat einerseits den Vorteil, dass dadurch der Messstation ein Laubbaum-ähnlicher Charakter verliehen wird. Andererseits haben sich derartige Kugel-förmige Elemente als recht wartungs- und reinigungsunaufwändig herausgestellt, da Regen von den Kugel-förmigen Elementen abfließen kann und dabei Verunreinigungen auf den Kugel-förmigen Elementen abspült. Weiterhin bieten die gerundeten Oberflächen der Kugel-förmigen Elemente nur wenig Halt für Wildvögel, wie zum Beispiel Tauben, so dass es zu geringeren Verunreinigungen durch Vogelkot kommt.

Die Kugel-förmigen Elemente können vollständig kugelförmig (im Sinne einer mathematischen Kugel) oder zum Beispiel auch kugelsegmentförmig sein, wobei die Kugelkalotte des Kugelsegments vorzugsweise nach außen weist. Die Kugel-förmigen Elemente sind hohl, um das Gewicht zu reduzieren. Weiterhin ermöglichen hohle Kugel-förmige Elemente, Komponenten wie zum Beispiel Messsensoren oder erfindungsgemäß Beleuchtungsmittel in den Kugel-förmigen Elementen anzuordnen. Die Kugel-förmigen Elemente bestehen vorzugsweise aus Kunststoff, da dadurch ein geringes Gewicht und eine gute Widerstandsfähigkeit der Kugeln gegenüber chemischen oder mechanischen Einwirkungen erreicht werden kann. Zur Abdichtung, insbesondere vor Feuchtigkeit, können die Kugel-förmigen Elemente beispielsweise einen abgedichteten, vorzugsweise verschraubbaren Boden aufweisen.

Es sind Beleuchtungsmittel vorgesehen und zur Beleuchtung der Kronen-artigen Struktur angeordnet. Erfindungsgemäß sind Beleuchtungsmittel vorgesehen und zur Beleuchtung mindestens eines, vorzugsweise mehrerer der Kugel-förmigen Elemente angeordnet. Zudem ist eine Steuereinrichtung vorgesehen, die dazu eingerichtet ist, die Beleuchtungsmittel anzusteuern. Als Beleuchtungsmittel kommen insbesondere LEDs oder auch LED-Folien in Betracht. Beispielsweise können eine oder mehrere LEDs in jeweils einem hohlen Kugel-förmigen Element angeordnet sein. Vorzugsweise sind die Beleuchtungsmittel so gewählt und/oder angeordnet, dass sich verschiedene Bereiche der Kronen-artigen Struktur in verschiedenen Farben beleuchten lassen. Insbesondere können die Beleuchtungsmittel so gewählt und/oder angeordnet sein, dass sich die einzelnen Kugel-förmigen Elemente in verschiedenen Farben beleuchten lassen. Die Kugel-förmigen Elemente sind zu diesem Zweck vorzugsweise zumindest teilweise transparent, um das Licht darin angeordneter Beleuchtungsmittel nach außen zu lassen.

Die Steuereinrichtung ist vorzugsweise derart eingerichtet, dass die Beleuchtung der Kronen-artigen Struktur, insbesondere der Kugel-förmigen Elemente tageszeitabhängig erfolgt. Vorzugsweise wird die Beleuchtungsstärke abends automatisch gedimmt. Auf diese Weise kann störende Lichtemission reduziert werden.

Erfindungsgemäß ist die Steuereinrichtung dazu eingerichtet, die Beleuchtungsmittel abhängig von den mit dem mindestens einen Messsensor gemessenen Messwerten zu steuern. Insbesondere können die Helligkeit und/oder die Farbe der Beleuchtungsmittel abhängig von den gemessenen Messwerten gesteuert werden. Beispielsweise kann die Kronen-artige Struktur mit den Beleuchtungsmitteln in einer ersten Farbe, zum Beispiel grün, erleuchtet werden, wenn der gemessene Wert in einem ersten vorgegebenen Bereich liegt, und in einer zweiten Farbe, zum Beispiel gelb oder rot, erleuchtet werden, wenn der gemessene Wert in einem zweiten vorgegebenen Bereich liegt. So kann die Kronen-artige Struktur zum Beispiel grün erleuchtet werden, wenn ein gemessener Feinstaubgehalt unter einem ersten Grenzwert liegt, gelb erleuchtet werden, wenn der Feinstaubgehalt zwischen einem ersten und einem zweiten Grenzwert liegt und rot erleuchten, wenn der Feinstaubgehalt über dem zweiten Grenzwert liegt. Auf diese Weise kann Passanten anhand der Farbe der Kronen-artigen Struktur die aktuelle Feinstaubbelastung der Umgebungsluft angezeigt werden. Gegenüber einer Anzeige auf einem Bildschirm hat die Beleuchtung der Kronen-artigen Struktur den Vorteil, dass viele Passanten gleichzeitig auch aus größerer Entfernung die Luftqualität unmittelbar und intuitiv erkennen können.

Die für die Steuerung der Beleuchtungsmittel zu verwendende Messgröße bzw. ein daraus berechneter Parameter, Farben und Grenzwerte sind vorzugsweise an der Steuereinrichtung einstellbar und können daher den Umgebungsgegebenheiten angepasst werden. Beispielsweise können für einen Luftkurort andere Grenzwerte gelten als für eine Hauptverkehrsstraße.

Die Steuerung der Beleuchtungsmittel kann auch abhängig von Messwerten mehrerer Messgrößen, zum Beispiel abhängig vom Feinstaubgehalt, vom Ozongehalt und/oder vom Stickoxidgehalt der Luft, durchgeführt werden. Beispielsweise können die Messwerte mehrerer Messgrößen in einen bestimmten Parameter für die Luftqualität umgerechnet werden und die Beleuchtungsmittel abhängig vom Wert dieses Parameters angesteuert werden. Auf diese Weise können auch komplexe Qualitätsparameter der Luftqualität in einfacher Weise für Passanten sichtbar gemacht werden.

Bei einer weiteren Ausführungsform weist die Messstation eine Schnittstelle zur Ausgabe mit mindestens einem Messsensor gemessener Messwerte, insbesondere der mit dem mindestens einen Messsensor gemessenen Messwerte auf. Beispielsweise kann die Messstation einen Bildschirm, insbesondere einen Touchscreen, aufweisen, zum Beispiel an der Stamm-artigen Struktur, über den sich die Messwerte oder aufbereitete Daten auf Basis der Messwerte anzeigen lassen. Auf diese Weise können Passanten sich zum Beispiel genauer über die Luftqualität informieren, zum Beispiel wenn die Beleuchtung der Kronen-artigen Struktur eine erhöhte Belastung anzeigt. Zusätzlich oder alternativ kann die Messstation auch eine digitale Datenschnittstelle, zum Beispiel zur Kommunikation an ein Netzwerk wie LAN oder WLAN oder an ein einzelnes Gerät (zum Beispiel über Bluetooth) aufweisen, so dass sich die Messwerte extern beobachten und auswerten lassen.

An der Messstation kann auch ein maschinenlesbarer Code, zum Beispiel ein QR-Code vorgesehen sein, in dem eine Kennung der Messstation oder ein Link zu einer Seite codiert ist, über die ein Nutzer Informationen über die Messstation oder von der Messstation gemessene Messwerte abrufen kann.

Bei einer weiteren Ausführungsform, ist die Steuereinrichtung dazu eingerichtet, über eine digitale Datenschnittstelle Daten aus einem Netzwerk abzurufen, beispielsweise aus dem Internet. Insbesondere kann die Steuereinrichtung dazu eingerichtet sein, Wetter- oder Verkehrsdaten abzurufen und diese über eine Schnittstelle wie zum Beispiel einen Bildschirm, auszugeben oder die Beleuchtungsmittel abhängig von den Wetter- oder Verkehrsdaten zu steuern. Beispielsweise können die Beleuchtungsmittel abhängig von externen Daten, wie zum Beispiel abgerufenen Wetter- oder Verkehrsdaten, und abhängig von gemessenen Messwerten gesteuert werden. So können ein Parameter für die Luftqualität abhängig von Messwerten für die Feinstaubkonzentration und Wetterdaten für die zu erwartenden Niederschläge oder Windgeschwindigkeiten bestimmt und die Beleuchtungsmittel abhängig vom Wert dieses Parameters angesteuert werden. Dies ermöglicht die Berücksichtigung vor Ort nicht unmittelbar messbarer Einflüsse, wie zum Beispiel der Wettervorhersage, bei der Auswertung der Luftqualität.

Bei einer weiteren Ausführungsform ist die Steuereinrichtung über eine digitale Datenschnittstelle mit mindestens einer anderen Messstation verbunden und sendet die gemessenen Messwerte an die mindestens eine andere Messstation oder empfängt von der mindestens einen anderen Messstation gemessene Messwerte. Auf diese Weise können mehrere, beispielsweise baugleiche Messstationen ein Messnetzwerk bilden und damit Messdaten aus einem größeren räumlichen Bereich auswerten und/oder anzeigen, beispielsweise über die Ansteuerung der Beleuchtungsmittel. Dadurch kann an den Messstationen ein Überblick über eine Gesamtlage der Luftqualität in einem bestimmten Bereich, zum Beispiel einer Stadt, abgerufen oder angezeigt werden. Weiterhin können die von dem Messnetzwerk gemessenen Messwerte vorzugsweise an einer zentralen Stelle, beispielsweise an einem zentralen Server, an den die einzelnen Messstationen ihre Messwerte übermitteln, abgerufen oder ausgewertet werden. Das Messnetzwerk muss nicht nur aus gleichartigen Messstationen wie der hier beschriebenen Messstation bestehen sondern kann auch konventionelle Messcontainer oder kompaktere Messstationen umfassen.

Da sich die Messstation aufgrund ihres Aufbaus vorteilhaft in das Stadtbild integrieren lässt, kann die Messstation bei einer weiteren Ausführungsform mit weiteren Fähigkeiten ausgestattet werden, die der Messstation einen weiteren Zusatznutzen verleihen. In Frage kommen hierzu insbesondere das Vorsehen eines WLAN-Hotspots, einer Steckdose zum Aufladen zum Beispiel von Kleingeräten wie Mobiltelefonen, einer Kraftfahrzeugladesäule, einer Notrufsäule und/oder von Sicherheits- oder Überwachungstechnik wie zum Beispiel Kameras oder Bewegungsmelder. Weiterhin kann die Messstation zu Werbe- oder Marketingzwecken genutzt werden, zum Beispiel indem entsprechende Inhalte auf einem an der Messstation vorgesehenen Bildschirm ausgegeben werden.

Bei einer Ausführungsform weist die Messstation eine Steuereinrichtung auf, die dazu eingerichtet ist, beim Messen einer schlechten Luftqualität bzw. einer hohen Belastung, zum Beispiel durch Feinstaub oder UV-Strahlung, eine Warnung auszugeben, beispielsweise indem die Kronen-artige Struktur mit den Beleuchtungsmitteln in einer bestimmten Farbe, zum Beispiel rot, angeleuchtet wird, eine Warnmeldung über einen vorgesehenen Lautsprecher oder einen vorgesehenen Bildschirm oder auch über eine elektrische Datenverbindung wie zum Beispiel über ein Netzwerk ausgegeben wird. Auf diese Weise können Personen um die Messstation unmittelbar vor einer lokalen schlechten Luftqualität bzw. hohen Belastung gewarnt werden.

Bei einer weiteren Ausführungsform ist die Steuereinrichtung dazu eingerichtet, einen ersten Teil der Beleuchtungsmittel abhängig von Messwerten einer ersten Messgröße für die Luftqualität, zum Beispiel der Feinstaubkonzentration, und einen zweiten Teil der Beleuchtungsmittel abhängig von Messwerten für eine zweite Messgröße für die Luftqualität, zum Beispiel der Ozonkonzentration, zu steuern. Beispielsweise können verschiedene Bereiche der Kronen-artigen Struktur abhängig von verschiedenen Messwerten bzw. daraus bestimmten Parametern beleuchtet werden. Dies ermöglicht es in einfacher Weise, Passanten über mehrere Messgrößen oder Parameter gleichzeitig zu informieren.

Bei einer weiteren Ausführungsform ist die Messstation modular aufgebaut. Dies ermöglicht einen einfacheren Transport der Messstation. Insbesondere können die Standbasis, die Stamm-artige Struktur und die Kronen-artige Struktur als separate Bauteile ausgebildet sein, die sich durch an den Bauteilen vorgesehene Verbindungsmittel zusammenfügen lassen.

Weitere Merkmale und Vorteile der Messstation ergeben sich aus der nachfolgenden Beschreibung von Ausführungsbeispielen, wobei auf die beigefügte Zeichnung Bezug genommen wird.

In der Zeichnung zeigen
- Fig. 1: ein Ausführungsbeispiel der Messstation in schematischer Ansicht,
- Fig. 2: die Messstation aus Fig. 1 in schematischer Schnittansicht,
- Fig. 3: ein weiteres Ausführungsbeispiel der Messstation in schematischer Schnittansicht und
- Fig. 4: die Messstation aus Fig. 1 in einer weiteren schematischen Schnittansicht.

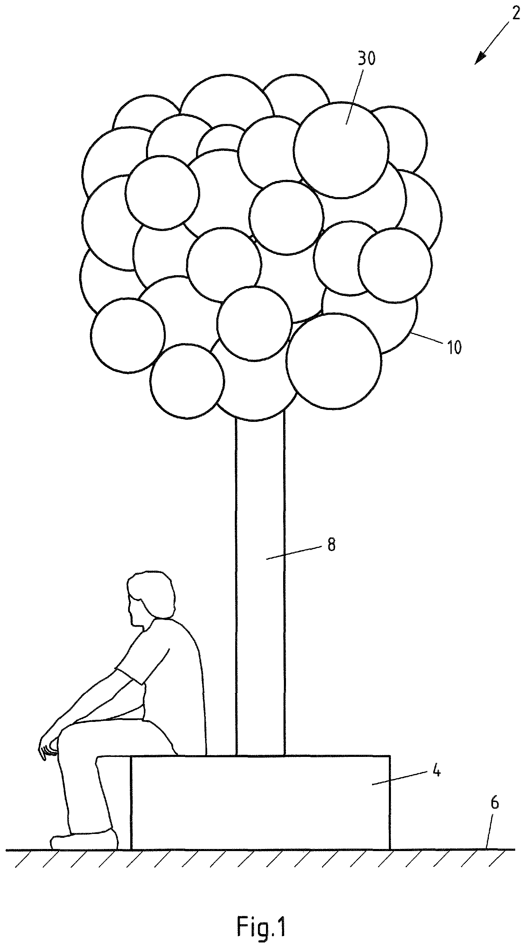

Figur 1 zeigt ein Ausführungsbeispiel der Messstation 2 in schematischer Seitenansicht. Die Messstation weist eine Standbasis 4 zum Aufstellen der Messstation 2 auf einem Untergrund 6, eine sich von der Standbasis 4 in die Höhe erstreckende Stamm-artige Struktur 8 und eine am oberen Ende der Stamm-artigen Struktur 8 angeordnete Kronen-artige Struktur 10 auf.

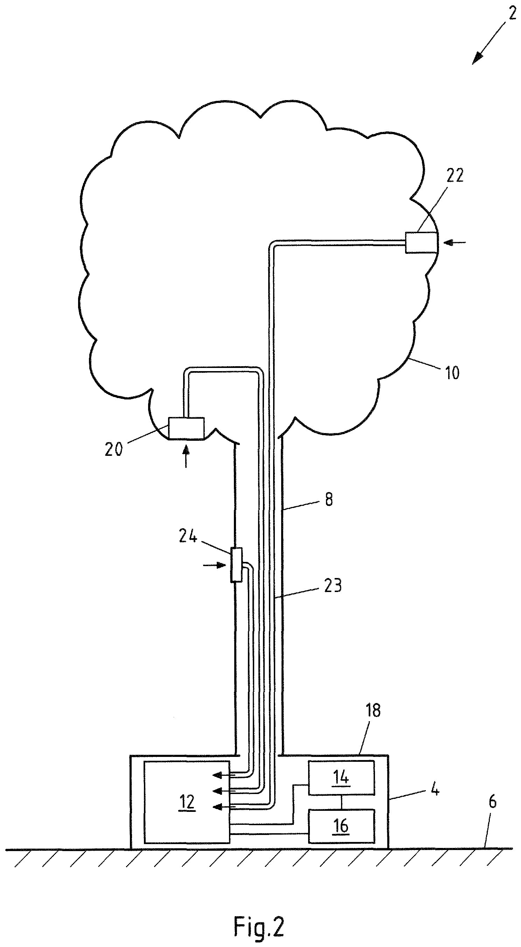

Figur 2 zeigt die Messstation 2 in schematischer Schnittansicht.

In die Messstation 2 ist ein Messsensor 12 integriert, mit dem Messwerte einer Messgröße für die Luftqualität, zum Beispiel für die Konzentration bestimmter Gase oder Feststoffe in der Luft, gemessen werden können. Der Messsensor 12 ist bei der Messstation 2 in der Standbasis 4 angeordnet, ebenso wie ein Energiespeicher 14 in Form einer Batterie zur Energieversorgung des Messsensors 12 und weiterer Komponenten der Messstation 2 und eine Steuereinrichtung 16.

Zum Schutz der in der Standbasis 4 angeordneten Komponenten weist die Standbasis 4 eine geschlossene, wasserdichte und robuste Hülle 18 auf, die die darin angeordneten Komponenten vor Witterung oder Vandalismus schützt. Die Standbasis kann zusätzlich Gewichte aufweisen, um die Stabilität der Messstation 2 zu gewährleisten.

Im Bereich der Kronen-artigen Struktur 10 sind mehrere Lufteinlässe 20, 22 vorgesehen, durch die Luft aus dem Bereich der Kronen-artigen Struktur 10 in das Innere der Messstation 2 gelangt. Durch vorgesehene Luftzuleitungen 23, die von den Lufteinlässen 20, 22 durch die Kronen-artige Struktur 10 und die Stamm-artige Struktur 8 bis zum Messsensor 12 verlaufen, gelangt die Luft zum Messsensor 12, so dass mit dem Messsensor 12 Messwerte einer Messgröße für die Luftqualität, insbesondere für Konzentrationen bestimmter gasförmiger Inhaltsstoffe (zum Beispiel CO, CO₂, NOₓ, O₃) oder fester Inhaltsstoffe (zum Beispiel Feinstaub), der Luft im Bereich der Kronen-artigen Struktur 10 gemessen werden können.

Um auch in anderen Höhen Messwerte von Messgrößen für die Luftqualität messen zu können, weist die Messstation 2 in Figur 2 einen weiteren Lufteinlass 24 im Bereich der Stamm-artigen Struktur auf. Auf diese Weise kann die Luftqualität in unterschiedlichen Höhen bestimmt werden.

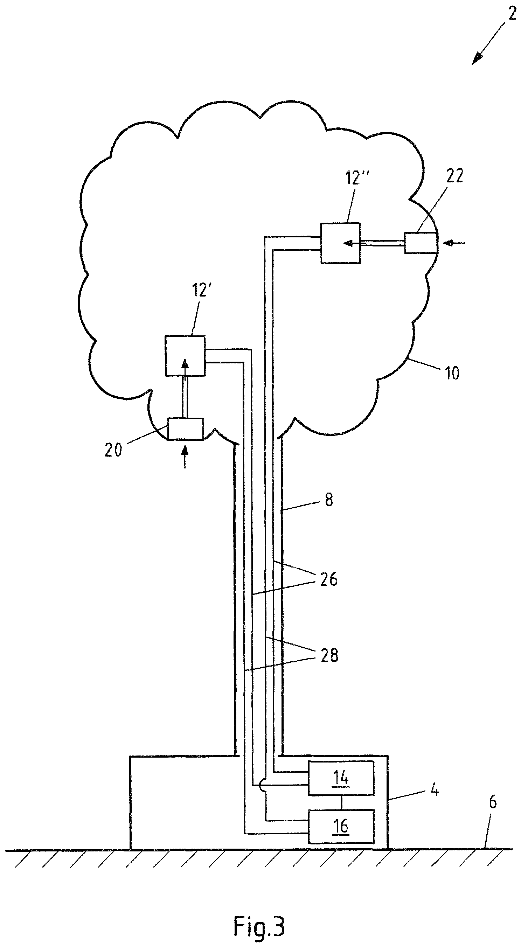

Figur 3 zeigt eine alternative Ausgestaltung der Messstation 2', bei der Messsensoren 12' und 12" in der Kronen-artigen Struktur 10 angeordnet sind. Zur Stromversorgung sind die Sensoren 12' und 12" mittels durch die Stamm-artige Struktur 8 verlaufender Stromleitungen 26 mit dem Energiespeicher 14 verbunden. Weiterhin sind die Sensoren 12' und 12" über Datenleitungen 28 mit der Steuereinrichtung 16 verbunden, so dass die mit den Sensoren 12', 12" gemessenen Werte zur Steuereinrichtung 16 übertragen und dort ausgewertet oder weiter verarbeitet werden können.

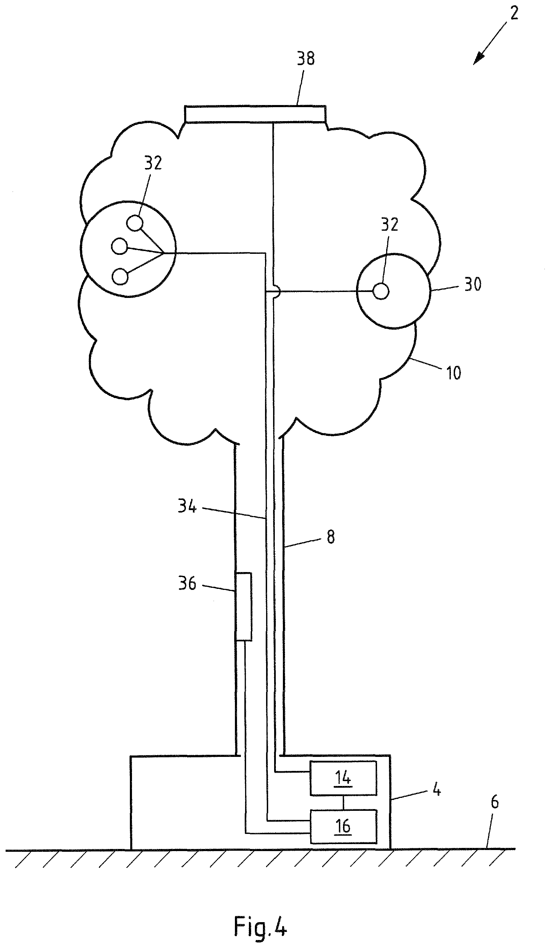

Figur 4 zeigt wiederum die Messstation 2 aus Figur 1 und 2 in schematischer Querschnittsansicht, wobei der Übersicht halber einige Komponenten aus Figur 2 ausgeblendet und dafür andere Komponenten dargestellt sind.

Wie in den Figuren 1 und 4 schematisch dargestellt, wird die Kronen-artige Struktur 10 durch miteinander verklebte kugel-förmige Elemente 30 in Form von Hohlkugeln oder Hohlkugelsegmenten gebildet und hat daher das Erscheinungsbild einer Laubbaum-Baumkrone. Die kugel-förmigen Elemente 30 bestehen vorzugsweise aus Kunststoff und sind zumindest teiltransparent, so dass sie von innen beleuchtet werden können. Zu diesem Zweck sind in den kugel-förmigen Elementen 30 Beleuchtungsmittel 32 in Form von Leuchtdioden angeordnet, die über Steuerleitungen 34 mit der Steuereinrichtung 16 verbunden sind und von dieser vorzugsweise einzeln angesteuert werden können. Fig. 4 zeigt die in den kugelförmigen Elementen 30 angeordneten Beleuchtungsmittel 32 exemplarisch an zwei kugelförmigen Elementen 30.

Bei den Leuchtdioden 32 kann es sich um einfarbige Leuchtdioden handeln oder auch um mehrfarbige Leuchtdioden, deren Farbe über die Steuereinrichtung 16 eingestellt werden kann.

Vorzugsweise ist die Steuereinrichtung 16 dazu eingerichtet, die Beleuchtungsmittel 32 abhängig von den von dem Messsensor 12 gemessenen Messwerten anzusteuern. Auf diese Weise kann die Kronen-artige Struktur 10 abhängig von der mit dem Messsensor 12 ermittelten Luftqualität beleuchtet werden. Handelt es sich bei dem Messsensor 12 zum Beispiel um einen Messsensor für die Feinstaubkonzentration in der Luft, so kann die Steuereinrichtung dazu eingerichtet sein, die Kronen-artige Struktur in einer ersten Farbe, zum Beispiel grün, erleuchten zu lassen, wenn sich der gemessene Feinstaubgehalt in einem ersten, zum Beispiel gesundheitlich unbedenklichen Bereich befindet, und in einer zweiten Farbe, zum Beispiel rot, erleuchten zu lassen, wenn sich der Feinstaubgehalt in einem zweiten, zum Beispiel erhöhten bzw. gesundheitlich bedenklichen Bereich befindet.

Die Beleuchtung der Kronen-artigen Struktur 10 kann auch abhängig von einer Kombination verschiedener Messwerte gesteuert werden. Weiterhin können unterschiedliche Bereiche der Kronen-artigen Struktur 10 in verschiedenen Farben beleuchtet werden. So ist zum Beispiel denkbar, dass ein erster Bereich der Kronen-artigen Struktur 10 abhängig von einer ersten Messgröße der Luftqualität, zum Beispiel der Os-Konzentration, und ein zweiter Bereich abhängig von einer zweiten Messgröße der Luftqualität, zum Beispiel der Feinstaubkonzentration, beleuchtet werden.

Durch eine solche Ansteuerung der Beleuchtungsmittel 32 erlaubt die Messstation 2 nicht nur die Messung der aktuellen Luftqualität, sondern zeigt diese für Passanten auch erkennbar an.

Um weitere Informationen über die gemessenen Messwerte und ggf. Auswertungen anzeigen zu können, kann an der Messstation, beispielsweise an der Stamm-artigen Struktur 8, ein Monitor 36 vorgesehen sein, der gesteuert von der Steuereinrichtung 16 verschiedene Informationen anzeigen kann. Weiterhin kann zur Stromversorgung ein Solarpanel 38 vorgesehen sein, dass mit dem Energiespeicher 14 verbunden ist und diesen auflädt.

Die Steuereinrichtung 16 kann dazu eingerichtet sein, Daten über eine digitale Schnittstelle zum Beispiel über LAN oder WLAN an ein Netzwerk oder zum Beispiel über Bluetooth an ein Mobilgerät zu übertragen. Insbesondere kann die Steuereinrichtung 16 an das Internet angeschlossen sein, um gemessene Daten an einen zentralen Server zu übermitteln.

Weiterhin kann die Messstation 2 mit anderen, insbesondere gleichartigen Messstationen zu einem Messnetzwerk zusammengeschlossen sein, in dem die einzelnen Messstationen 2 die jeweils lokal gemessenen Messwerte austauschen oder an einen zentralen Server übermitteln, so dass die Luftqualität über einen größeren Bereich, über den das Messnetzwerk verteilt ist, insgesamt ausgewertet werden kann.

Zur Nutzung der Messstation 2 als öffentliches Möbelstück ist die Standbasis 4 wie in Fig. 1 dargestellt vorzugsweise derart dimensioniert, dass sie als Sitzplatz dienen kann. Der Abstand zwischen der Oberkante der Standbasis 4 und der Unterkante der Kronen-artigen Struktur 10 beträgt vorzugsweise mindestens 2 m. Auf diese Weise kann ein erwachsener Mensch bequem auf der Standbasis 4 stehen oder darunter sitzen, ohne an die Kronen-artige Struktur 10 zu stoßen. Weiterhin kann auf diese Weise Vandalismus an der Kronen-artigen Struktur vorgebeugt werden.

## Patentansprüche

1. Messstation (2, 2'), eingerichtet zum Messen der Luftqualität, für die Freiluftaufstellung im städtischen Bereich
- mit einer Standbasis (4) zum Aufstellen der Messstation (2, 2') auf einem Untergrund (6),
- mit einer Stamm-artigen Struktur (8), die sich von der Standbasis (4) in die Höhe erstreckt, und
- mit einer Kronen-artigen Struktur (10) am oberen Ende der Stamm-artigen Struktur (8),
- wobei mindestens ein Messsensor (12, 12', 12") derart in die Messstation (2, 2') integriert ist, dass mit dem Messsensor (12, 12', 12") Messwerte einer Messgröße für die Luftqualität im Bereich der Kronen-artigen Struktur (10) messbar sind, **dadurch gekennzeichnet,**
- **dass** die Kronen-artige Struktur (10) mehrere hohle Kugel-förmige Elemente (30) aufweist, die die äußere Form der Kronen-artigen Struktur (10) vorgeben,
- **dass** Beleuchtungsmittel (32) vorgesehen und zur Beleuchtung mindestens eines, vorzugsweise mehrerer der Kugel-förmigen Elemente (30) angeordnet sind, wobei die Beleuchtungsmittel (32) in den hohlen Kugel-förmigen Elementen angeordnet sind, und
- **dass** eine Steuereinrichtung (16) vorgesehen ist, die dazu eingerichtet ist, die Beleuchtungsmittel (32) abhängig von den mit dem mindestens einen Messsensor (12, 12', 12") gemessenen Messwerten zu steuern.

2. Messstation nach Anspruch 1,
**dadurch gekennzeichnet, dass** der Messsensor (12, 12', 12") in die Standbasis (4) integriert ist und eine von der Kronen-artigen Struktur (10) durch die Stamm-artige Struktur (8) zur Standbasis (4) verlaufende Luftzuleitung (23) zur Versorgung des Messsensors (12, 12', 12") mit Luft aus dem Bereich der Kronen-artigen Struktur (10) vorgesehen ist.

3. Messstation nach Anspruch 1 oder 2,
**dadurch gekennzeichnet, dass** ein Energiespeicher (14) zur Versorgung der Messstation (2, 2') mit elektrischer Energie in die Standbasis (4) integriert ist, insbesondere eine Batterie.

4. Messstation nach einem der Ansprüche 1 bis 3,
**dadurch gekennzeichnet, dass** die Standbasis (4) eine wasserundurchlässige Hülle (18) aufweist.

5. Messstation nach einem der Ansprüche 1 bis 4,
**dadurch gekennzeichnet, dass** die Standbasis (4) eine Sitzgelegenheit aufweist, insbesondere als Sitzbank.

6. Messstation nach einem der Ansprüche 1 bis 5,
**dadurch gekennzeichnet, dass** an der Stamm-artigen Struktur (8) ein Lufteinlass (24) vorgesehen ist und mindestens ein Messsensor (12, 12', 12") derart in die Messstation integriert ist, dass mit dem Messsensor (12, 12', 12") Messwerte einer Messgröße für die Luftqualität der durch den Lufteinlass (24) einströmenden Luft messbar sind.

7. Messstation nach einem der Ansprüche 1 bis 6,
**dadurch gekennzeichnet, dass** die Kronen-artige Struktur (10) mehrere Kugel-förmige Elemente (30) von verschiedener Größe aufweist, die die äußere Form der Kronen-artigen Struktur (10) vorgeben.

8. Messstation nach einem der Ansprüche 1 bis 7,
**dadurch gekennzeichnet, dass** die Messstation (2, 2') eine Schnittstelle (36) zur Ausgabe der mit dem mindestens einen Messsensor (12, 12', 12") gemessenen Messwerte aufweist.

## Claims

1. Measuring station (2, 2'), configured to measure air quality, for outdoor installation in urban areas,
- having a stand base (4) for setting up the measuring station (2, 2') on a ground (6),
- having a trunk-like structure (8) extending upwards from the stand base (4), and
- having a treetop-like structure (10) at the upper end of the trunk-like structure (8),
- wherein at least one measuring sensor (12, 12', 12") is integrated into the measuring station (2, 2') in such a way that measurement values of a measuring quantity for the air quality in the region of the treetop-like structure (10) can be measured with the measuring sensor (12, 12', 12"),
**characterised**
- **in that** the treetop-like structure (10) comprises a plurality of hollow spherical elements (30) which define the outer shape of the treetop-like structure (10),
- **in that** illumination means (32) are provided and arranged for illuminating at least one, preferably several, of the spherical elements (30), the illumination means (32) being arranged in the hollow spherical elements, and
- **in that** a control device (16) is provided, which is configured to control the illumination means (32) as a function of the measurement values measured by the at least one measuring sensor (12, 12', 12").

2. Measuring station according to claim 1,
**characterised in that** the measuring sensor (12, 12', 12") is integrated into the stand base (4) and an air supply line (23) extending from the treetop-like structure (10) through the trunk-like structure (8) to the stand base (4) is provided for supplying the measuring sensor (12, 12', 12") with air from the region of the treetop-like structure (10).

3. Measuring station according to claim 1 or 2,
**characterised in that** an energy storage (14) for supplying the measuring station (2, 2') with electrical energy is integrated into the stand base (4), in particular a battery.

4. Measuring station according to one of claims 1 to 3,
**characterised in that** the stand base (4) has a water-impermeable cover (18).

5. Measuring station according to one of claims 1 to 4,
**characterised in that** the stand base (4) has a seat, in particular as a bench.

6. Measuring station according to one of claims 1 to 5,
**characterised in that** an air inlet (24) is provided on the trunk-like structure (8) and at least one measuring sensor (12, 12', 12") is integrated into the measuring station in such a way that measurement values of a measuring quantity for the air quality of the air flowing in through the air inlet (24) can be measured with the measuring sensor (12, 12', 12").

7. Measuring station according to one of claims 1 to 6,
**characterised in that** the treetop-like structure (10) has a plurality of spherical elements (30) of different sizes, which define the outer shape of the treetop-like structure (10).

8. Measuring station according to one of claims 1 to 7,
**characterised in that** the measuring station (2, 2') has an interface (36) for outputting the measurement values measured with the at least one measuring sensor (12, 12', 12").

## Revendications

1. Station de mesure (2, 2'), configurée pour mesurer la qualité de l'air, pour installation en plein air en zone urbaine
- avec une base d'appui (4) pour poser la station de mesure (2, 2') sur un sol (6),
- avec une structure de type de tronc (8) qui s'étend en hauteur à partir de la base d'appui (4), et
- avec une structure de type couronne (10) à l'extrémité supérieure de la structure de type de tronc (8),
- où au moins un capteur de mesure (12, 12', 12") est intégré dans la station de mesure (2, 2') de telle sorte qu'avec le capteur de mesure (12, 12', 12"), des valeurs de mesure d'une grandeur de mesure sont mesurables pour la qualité de l'air dans la zone de la structure de type couronne (10),
**caractérisée**
- **en ce que** la structure de type couronne (10) a une pluralité d'éléments sphériques creux (30) qui définissent la forme extérieure de la structure de type couronne (10),
- **en ce que** des moyens d'éclairage (32) sont prévus et agencés pour éclairer au moins un, de préférence plusieurs, des éléments sphériques (30), les moyens d'éclairage (32) étant agencés dans les éléments sphériques creux, et
- **en ce qu'**un dispositif de commande (16) configuré pour commander les moyens d'éclairage (32) en fonction des valeurs de mesure mesurées par l'au moins un capteur de mesure (12, 12', 12") est prévu.

2. Station de mesure selon la revendication 1,
**caractérisée en ce que** le capteur de mesure (12, 12', 12") est intégré dans la base d'appui (4) et qu'une conduite d'alimentation en air (23) s'étendant de la structure de type couronne (10) à travers la structure de type de tronc (8) vers la base d'appui (4) est prévue pour alimenter le capteur de mesure (12, 12', 12") en air provenant de la zone de la structure de type couronne (10).

3. Station de mesure selon la revendication 1 ou 2,
**caractérisée en ce qu'**un accumulateur d'énergie (14) pour l'alimentation de la station de mesure (2, 2') en énergie électrique est intégré dans la base d'appui (4), en particulier une batterie.

4. Station de mesure selon l'une des revendications 1 à 3,
**caractérisée en ce que** la base d'appui (4) comporte une enveloppe (18) imperméable à l'eau.

5. Station de mesure selon l'une des revendications 1 à 4,
**caractérisée en ce que** la base d'appui (4) présente un siège, en particulier sous forme de banc.

6. Station de mesure selon l'une des revendications 1 à 5,
**caractérisée en ce qu'**une entrée d'air (24) est prévue au niveau de la structure de type de tronc (8) et qu'au moins un capteur de mesure (12, 12', 12") est intégré dans la station de mesure de telle sorte qu'avec le capteur de mesure (12, 12', 12"), des valeurs de mesure d'une grandeur de mesure pour la qualité de l'air affluant par l'entrée d'air (24) sont mesurables.

7. Station de mesure selon l'une quelconque des revendications 1 à 6,
**caractérisée en ce que** la structure de type couronne (10) comprend une pluralité d'éléments sphériques (30) de différentes tailles qui définissent la forme extérieure de la structure de type couronne (10).

8. Station de mesure selon l'une des revendications 1 à 7,
**caractérisée en ce que** la station de mesure (2, 2') présente une interface (36) pour mise à disposition des valeurs de mesure mesurées par l'au moins un capteur de mesure (12, 12', 12").
